# EUROPEAN PATENT APPLICATION

(11) **EP 2 698 198 A1**
(43) Date of publication of application: **19.02.2014**
(21) Application number: 12005876.3
(22) Date of filing: 14.08.2012
(51) Int. Cl.: B01J 29/40, B01J 29/42, B01J 29/48, B01J 37/06, C07C 2/24

(54) **Method for pre-treating a catalyst composition with water or alcohol vapour in nitrogen gas at temperatures below their boiling point**

(71) Applicant: Saudi Basic Industries Corporation, Riyadh 11422 (SA)
(72) Inventor: Jana, Suman Kumar, Manjusar 391775 District Baroda (IN)
(74) Representative: Modiano, Micaela Nadia

(57) **Abstract**

The present invention relates to a method for pre-treating a catalyst composition comprising contacting a medium pore aluminosilicate zeolite with an inert gas comprising water vapour or alcohol vapour at a temperature between 30 °C and the boiling temperature of water or the alcohol.

## Description

The present invention relates to a method for pre-treating a catalyst composition. The invention further relates to a process for the dimerization of olefins or a mixture of olefins and paraffins using the pre-treated catalyst composition.

A variety of octane boosters is on the market. Currently, the most popular octane booster is methyl tertiary butyl ether (MTBE). However, because of its hazardous nature, it is predicted that the worldwide consumption of MTBE will gradually decrease. A promising candidate for replacing MTBE is isooctane. Isooctane has similar properties to MTBE, exhibits high octane number and also has very good environmental properties.

Isooctane is typically produced by dimerization of isobutene followed by hydrogenation. A few technologies are available for commercialization for the selective production of isooctenes from isobutene based on solid macro-porous acidic resin or phosphoric acid catalyst. These technologies are based on solid macro-porous acidic resin or phosphoric acid catalyst and require additive(s) and/or hydrating agent(s) together with the catalyst to obtain isooctenes selectively. However, the presence of additive(s)/hydrating agent(s) in the feed stream contributes to the formation of some amount of unwanted product in the product stream.

Catalysis Today, 100 (2005) 463 discloses catalyst H-ZSM-5 (having Si/Al ratios of 15 and 25) for isobutene dimerization under liquid-phase conditions (40°C, 10 bar pressure and n-butane as diluent). Although the catalyst shows high initial conversion, it deactivates very rapidly. The initial conversion of ∼ 65% drops down to < 2% within 1 h of reaction, hence it is not suitable for industrial scale dimerization of isobutene.

EP 1167326 A1 discloses the dimerization of isobutene using a catalyst system comprising a zeolite with TON-type structure. The catalyst was unable to show the desired product selectivity for a long time. The dimer selectivity decreased from 72% to 54% within 4 h of reaction. The catalyst also got deactivated fast. Isobutene conversion decreased from 98% to 84% within 4 h of reaction.

WO 2004/080935 A1 discloses medium pore zeolites. H-ZSM-5, H-ZSM-22, H-ZSM-23, Ferrierite having Al content ∼ 0.1 to 5 wt% are reported for isobutene dimerization under high pressure reaction conditions. However, these catalysts deactivate fast and hence require frequent regeneration for long reaction process.

WO09027582 discloses a process for oligomerizing olefinic, lower hydrocarbons using an acidic catalyst selected from the group of natural and synthetic zeolites or from the mesoporous aluminosilicates. Zeolite is selected from the group consisting of ZSM-5, ZSM-22, ZSM-23, ferrierite and ion-exchanged zeolites prepared therefrom.

WO200480935 discloses a process for dimerizing lower, olefinic hydrocarbons with a medium pore zeolite under process conditions allowing selective dimerization. The olefinic hydrocarbon feedstock is contacted with an acid catalyst at conditions in which at least a part of the olefins dimerizes. The effluent from the reaction zone is conducted to the separation zone where dimerized reaction product is separated from said effluent.

US3325465 discloses a process for dimerizing isobutene using 13X molecular sieve wherein the 95.9% of sodium ion is counter ion exchanged with cobalt ion.

The major disadvantages of the known prior art are low isooctene selectivity and low catalyst stability. Also the use of ion-exchange resin catalysts requires cumbersome catalyst regeneration, which leads to the generation of more effluents.

It is an object of the present invention to provide an improved catalyst. It is a further object of the present invention to provide an improved process for the dimerization of olefins or a mixture of olefins and paraffins.

Accordingly, the present invention provides a method for pre-treating a catalyst composition comprising contacting a medium pore aluminosilicate zeolite with an inert gas comprising water vapour or alcohol vapour at a temperature between 30 °C and the boiling temperature of water or the alcohol.

According to the pre-treatment method of the present invention, the relatively strong acid sites of zeolite catalyst is reduced through control adsorption of water or alcohol molecule on the acidic sites. This results in that the severity of the dimerization reaction is minimized. The pre-treatment of the catalyst with an inert gas comprising water or alcohol vapour hence leads to a catalyst with a higher selectivity towards dimers, e.g. contacting isobutene with the pre-treated catalyst composition will result in a high selectivity towards isooctene (2,4,4-Trimethyl pentene). It is also advantageous that the feed stream does not require the presence of additive(s)/hydrating agent(s) which leads to the production of unwanted product(s).

The contact temperature of the zeolite with the inert gas comprising water vapour or alcohol vapour is preferably 30-90 °C, more preferably 40-70 °C.

The inert gas is preferably saturated with water vapour or alcohol vapour.

The alcohol is preferably selected from the group consisting of 1-propanol, isopropanol, isobutanol and tertiary butanol or a mixture thereof.

The inert gas is preferably selected from the group consisting of nitrogen, helium and argon or a mixture thereof.

### Optional contacting with dry gas

The pre-treatment method may further comprise the step of contacting the zeolite with an inert gas which does not comprise water vapour or alcohol vapour (hereinafter sometimes referred as "dry inert gas" or "dry gas") before and/or after the contacting step with the inert gas comprising water or alcohol vapour. The contacting step with the dry inert gas may be performed at the same temperature as or different temperature from the temperature of the contacting step with the inert gas comprising water vapour or alcohol. For example, the contacting step with the dry gas may be performed at 30 to 350 °C. The duration of the contacting step with the dry gas may e.g. be 0.1 to 3 hours.

### Contacting with dry gas before contacting with the moist gas

The contacting step with the dry inert gas before the contacting step with the inert gas comprising water or alcohol vapour is preferably performed at a temperature between 100 to 300 °C. Contacting the catalyst composition with the dry inert gas at a temperature between 100 to 300 °C before contacting with the inert gas comprising water or alcohol vapour helps quick removal of pre-adsorbed gases from the catalyst, resulting in cleaning the catalyst surface.

The contacting step with the dry inert gas before the contacting step with the inert gas comprising water or alcohol vapour may e.g. be 0.3 to 1.8 hours. It was found that this range is suitable for removing pre-adsorbed gases from the catalyst.

### Contacting with the moist gas

The contacting step with the inert gas comprising water or alcohol vapour is performed at a temperature between 30 °C and the boiling temperature of water or the alcohol, preferably 30-90 °C, more preferably 40-70 °C.

The contacting step with the inert gas comprising water or alcohol vapour is preferably performed for 0.2 to 2.0 hours. It was found that this range results in a combination of good isobutene conversion rate and good selectivity. Particularly preferred is contacting for 0.4 to 1.0 hours, which results in a combination of good conversion rate and very high selectivity towards dimer.

### Contacting with dry gas after contacting with the moist gas

The contacting step with the dry inert gas after the contacting step with the inert gas comprising water or alcohol vapour is preferably performed at a temperature between 50 °C and the boiling temperature of water or the alcohol Particularly preferred is the contacting at the boiling temperature of water or the alcohol or at a temperature at most 10 °C lower than the boiling temperature of water or the alcohol, which results in a combination of good conversion and very high selectivity towards dimer.

The contacting step with the dry inert gas after the contacting step with the inert gas comprising water or alcohol vapour may e.g. be 0.1 to 1.5 hours.. Particularly preferred is contacting for 0.5 to 1.0 hours, which results in a combination of good conversion and very high selectivity towards dimer.

Particularly preferred embodiments include a pre-treatment method comprising contacting with the dry gas at 300 °C for 1 h, subsequently contacting with the moist gas at 50 °C for 1 h, and subsequently contacting with the dry gas at the boiling temperature of water or the alcohol for 0.5 h.

### Zeolite

The zeolite used in the present invention is crystalline materials. Crystalline materials are preferred because of their regular pore size and channelling framework structures.

As used herein, the term "zeolite" or "aluminosilicate zeolite" relates to an aluminosilicate molecular sieve. These inorganic porous materials are well known to the skilled person. An overview of their characteristics is for example provided by the chapter on Molecular Sieves in Kirk-Othmer Encyclopedia of Chemical Technology, Volume 16, p 811-853; in Atlas of Zeolite Framework Types, 5th edition, (Elsevier, 2001).

Aluminosilicate zeolites are generally characterized by the Si/Al ratio of the framework. This ratio may vary widely in the catalyst composition used in the method according to the invention. Preferably, the silicon to aluminium (Si:Al) molar ratio of the zeolite is from about 5 to 1000, more preferably from about 8 to 500.

More preferably, the Si:Al molar ratio of the zeolite is 10-100, preferably 30-60 and most preferably 40-50. This molar ratio of the zeolite is very useful as the zeolite with Si/Al ratio in this range contains moderate acidity and assists in promoting acid catalyzed isobutene dimerization process selectively. This is achieved mostly by preventing unwanted oligomerization reaction through suppressing the severity of the reaction.

Any aluminosilicate that shows activity in the dimerization of olefins may be applied. Examples of suitable materials include the mordenite framework inverted (MFI) and other zeolite structures known to the skilled person, for example MEL, MWW, BEA, MOR, LTL and MTT type. Preferred materials are those known as ZSM-5, ZSM-11, ZSM-8, ZSM-12, ZSM-22, ZSM-23, ZSM-35, ZSM-38, and beta. Most preferably the zeolite is a MFI type zeolite, for example a ZSM-5 zeolite.

The term "medium pore zeolite" is commonly used in the field of zeolite catalyst compositions. A medium pore size zeolite is a zeolite having a pore size of 5-6 Å. Preferably, the zeolite has a pore size of 5-6 Å, more preferably a pore size of 5.2-5.8 Å. Medium pore size zeolites are 10-ring zeolites. i.e. the pore is formed by a ring consisting of 10 SiO₄ tetrahedra. Zeolites of the 8-ring structure type are called small pore size zeolites; and those of the 12-ring structure type, like for example beta zeolite, are referred to as large pore sized. In the above cited Altlas of Zeolite Framework Types, various zeolites are listed based on ring structure. Preferably, the zeolite is a medium pore size aluminosilicate zeolite.

The zeolite used in the present invention may be dealuminated. Means and methods to obtain dealuminated zeolite are well known in the art and include, but are not limited to the acid leaching technique; see e.g. Post-synthesis Modification I; Molecular Sieves, Volume 3; Eds. H. G. Karge, J. Weitkamp; Year (2002); Pages 204-255. Preferably, the zeolite is a dealuminated zeolite having a SiO₂ to Al₂O₃ molar ratio of 10 to 200, for improving the performance/stability of the catalyst composition. Means and methods for quantifying the SiO₂ to Al₂O₃ molar ratio of a dealuminated zeolite are well known in the art and include, but are not limited to AAS (Atomic Absorption Spectrometer) or ICP (Inductively Coupled Plasma Spectrometry) analysis.

The zeolite used in the present invention is in the hydrogen form: i.e. having at least a portion of the original cations associated therewith replaced by hydrogen. Methods to convert an aluminosilicate zeolite to the hydrogen form are well known in the art. One method involves base-exchange using ammonium salts followed by calcination.

The zeolite used in the present invention may comprise up to 5 wt-% of one or more elements selected from Groups 6 and 9 of the Periodic Table, i.e. chromium, molybdenum, tungsten, seaborgium, cobalt, rhodium, iridium and meitnerium. This results in an even higher selectivity towards isooctene. Preferably, said one or more elements are selected from the group consisting of molybdenum, tungsten, cobalt and rhodium.

The amount of these elements in the zeolite may e.g. be at most 5 wt-%, 4 wt-%, 3-wt%, 2-wt%, 1.5 wt%, 1.25 wt%, 1.1 wt% or 1.0 wt%. The amount of these elements in the zeolite may e.g. be at least 0.05 wt%, 0.1 wt%, 0.25 wt% or 0.5 wt%.

These elements may be present in the zeolite structure as framework or non-framework element; as counter ion in the zeolite; on its surface, e.g. in the form of metal oxides; or be present in a combination of these forms.

Preferably, the catalyst used in present invention has the framework structure of ZSM-5 with Bronsted acid sites provided by tetrahedrally coordinated aluminium in the framework structure and Lewis and/or Bronsted acid sites provided by elements from Groups 6 and 9 of the Periodic table in the framework structure.

The zeolite catalyst used in the present invention can be prepared by suitable methods of preparing and modifying zeolites as well known to the skilled person; including for example impregnation, calcination, steam and/or other thermal treatment steps. Such methods are disclosed for instance in documents US 7186872B2; US4822939 and US4180689 hereby incorporated by reference. The zeolite catalyst used in the present process can also be made by ion exchange technique, sonification technique, precipitation technique, which are all well known to the skilled person.

The catalyst composition comprises a zeolite catalyst as described above. The catalyst composition may consist of the zeolite catalyst as described above, or the catalyst composition may comprise further components such as diluents or binders or other support materials. Preferably these further components do not negatively affect the catalytic performance of the catalyst composition of the invention. Such components are known to the skilled person.

For example, the catalyst composition of the invention may further comprise a non- acidic inert diluent. Preferably the non-acidic inert diluent is silica.

Examples of suitable binder materials include metal oxides, mixed metal oxides, clays, metal carbides and metal oxide hydroxides. The metal oxide or the mixed metal oxides may be chosen from the group of metal oxides comprising for example, oxides of magnesium, aluminium, titanium, zirconium and silicon. The clay may be, but is not limited to, kaolin, montmorillonite or betonite. Metal carbides suitable for use in the composition of the invention are, for example, molybdenum carbide and silicon carbide. The metal oxide hydroxide may be feroxyhyte or Goethite, or more preferably boehmite.

The binder may be present in the composition according to the invention in for example at least 5 wt %, for example at least 10 wt%, for example at least 20 wt %, for example at least 30 wt %, for example at least 40 wt %, for example at least 50% and/or for example at most 5 wt %, for example at most 10 wt%, for example at most 20 wt %, for example at most 30 wt %, for example at most 40 wt %, for example at most 50 wt% with respect to the total catalyst composition.

If the zeolite catalyst composition is to contain a binder, such catalyst composition can be obtained, for example, by mixing the zeolite and a binder in a liquid, and forming the mixture into shapes, like pellets or tablets, applying methods known to the skilled person.

A further aspect of the present invention provides the pre-treated catalyst obtainable by the method according to the present invention.

A further aspect of the present invention provides a process for the dimerization of olefins or a mixture of olefins and paraffins, said olefins and paraffins having between 2 to 10 carbon atoms, preferably 2 to 8 carbon atoms, comprising:
(a) pre-treating a catalyst composition according to the method according to the present invention; and
(b) contacting the treated catalyst composition with a feedstream comprising said olefins or a mixture of olefins and paraffins.

A high selectivity to dimer was achieved. Catalyst was found to be stable.

Preferably, the molar ratio of the olefins to paraffins in the feedstream is 1:0.1-10, more preferably 1:0.2-5, and most preferably 1:0.5-2.

Preferably, the feedstream comprises isobutene and isobutane.

The feed stream may further contain one or more diluents, the concentration of which may vary over wide ranges; preferably the feed stream comprises 10-90 vol % of a feed diluent. Examples of suitable diluents include helium, nitrogen, carbon dioxide, and water.

The step of contacting the feed stream with the treated catalyst composition can be performed in any suitable reactor, as known to a skilled man, for example in a fixed bed, a fluidized bed, or any other circulating or moving bed reactor. With reactor is meant a device for containing and controlling a chemical reaction, in this case dimerization reaction of olefins such as isobutene .

The step of contacting the feed stream with the treated catalyst composition is performed at olefin dimerization conditions. These conditions are known from the prior art. A higher temperature generally enhances conversion and formation of oligomers. However, higher temperatures may induce side-reactions or promote deactivation of the catalyst, Therefore, the contacting step is preferably performed at a temperature of 40-80 °C.

Suitable pressures to conduct the contacting step are from between 1-2.5 MPa.

The flow rate at which the feed stream is fed to the reactor may vary widely, but is preferably such that a liquid hourly space velocity (LHSV) results of about 0.1 - 100 h⁻¹, more preferably LHSV is about 0.5 -50 h⁻¹, or 1 - 20 h⁻¹ or most preferably 7.5-15 h⁻¹. The LHSV may be preferably at least 0.1 h⁻¹, for example at least 10 h⁻¹, for example at least 20 h⁻¹, for example at least 30 h⁻¹ and/or for example at most 1 h⁻¹, for example at most 10 h⁻¹, for example at most 20 h⁻¹, for example at most 30 h⁻¹, for example at most 40 h⁻¹, for example at most 50 h⁻¹. LHSV is the ratio of the rate at which the feed stream is fed to the reactor (in volume per hour) divided by the weight of catalyst composition in a reactor; and is thus inversely related to contact time. By contact time is meant the period of time during which the feedstream is in contact with the catalyst composition.

The LHSV indicates that there is a certain rate at which the feedstream is fed to the reactor. The total length of time in which the feedstream is fed to the reactor is known as the "Time-on-Stream (TOS)." The TOS may be for example at least 2 hours, for example at least 10 hours, for example at least 50 hours, for example at least 100 hours and/or for example at most 2 hours, for example at most 10 hours, for example at most 50 hours, for example at most 100 hours. For example the TOS for a catalyst composition according to the invention during which time the catalyst composition maintains its activity in terms of a high conversion and high selectivity for isooctene, ranges from for example 10 to 100 hours, for example from 15 to 50 hours.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims,

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention will now be further illustrated with below described experiments.

### Comparative experiment 1

Various pre-treated catalysts were used for isobutene dimerization, as shown in Table 1 .

Pre-calcined samples as shown in Table 1 were pre-treated at 100°C for 1 h under 20 ml/min of flowing dry N₂.

A feed of isobutene diluted with isobutane (50 : 50 weight ratio) was contacted with the pre-treated catalysts at 50 °C and 20 bar for 2.5 hours.

**Table 1**

| Catalysts (Si/Al ratio) | Isobutene conversion / % | Selectivity / % | | | |
|---|---|---|---|---|---|
| | | C₈ | | C₁₂ | C_{16&+} |
| | | 2,4,4-Trimethyl pentene | Others | | |
| H-ZSM-5 (27.5) | 99.8 | 13.4 | 1.2 | 43.0 | 42.4 |
| H-ZSM-5 (45) | 37.4 | 87.9 | 0.0 | 4.3 | 7.8 |
| H-Mordenite (20) | 98.0 | 28.0 | 8.3 | 49.5 | 14.2 |
| H-Beta (20.5) | 93.5 | 16.1 | 0.2 | 62.5 | 21.2 |
| H-Y (6) | 77.3 | 12.9 | 0.5 | 27.6 | 59.0 |
| H-SAPO-5 (0.4) | 10.3 | 79.0 | 0 | 17.5 | 3.5 |
| H-SAPO-11 (0.1) | 0.4 | 100 | 0 | 0 | 0 |

### Comparative experiment 2

Results of isobutene dimerization over H-ZSM-5 zeolite having different Si/Al ratios are shown in Table 2. The pre-treatment of the catalyst, the feed and the dimerization conditions were the same as in comparative experiment 1.

**Table 2**

| Si/Al mole ratio | Isobutene conversion /% | Selectivity / % | | | |
|---|---|---|---|---|---|
| | | C₈ | | C₁₂ | C_{16&+} |
| | | 2,4,4-Trimethyl pentene | Others | | |
| 27.5 | 99.8 | 13.4 | 1.2 | 43.0 | 42.4 |
| 35 | 61.2 | 81.5 | 0.0 | 11.7 | 6.8 |
| 45 | 37.4 | 87.9 | 0.0 | 4.3 | 7.8 |
| 100 | 14.6 | 93.9 | 0.0 | 6.1 | 0.0 |

### Comparative experiment 3

H-ZSM-5 zeolite (Si/Al ratio 45) catalysts pre-treated at different conditions were used for isobutene dimerization.

Pre-calcined samples were pre-treated at various temperatures shown in Table 3 for 1 h under 20 ml/min of flowing dry N₂.

The feed and the dimerization conditions were the same as in comparative experiment 1.

**Table 3**

| Catalysts pre-treatment temperature / °C | Isobutene conversion /% | Selectivity / % | | | |
|---|---|---|---|---|---|
| | | C₈ | | C₁₂ | C_{16&+} |
| | | 2,4,4-Trimethyl pentene | Others | | |
| 50 | 35.9 | 89.3 | 1.0 | 8.9 | 0.8 |
| 100 | 37.4 | 87.9 | 0.0 | 4.3 | 7.8 |
| 120 | 72.1 | 81.3 | 0.4 | 16.5 | 1.8 |
| 150 | 94.6 | 35.7 | 9.4 | 48.2 | 6.7 |
| 200 | 98.4 | 10.7 | 32.7 | 53.9 | 2.7 |
| 300 | 99.5 | 3.4 | 5.7 | 28.3 | 62.6 |

### Example 1

Various pre-treated catalysts shown in Table 4 was used for isobutene dimerization.

Pre-calcined samples as shown in Table 4 was pre-treated at 300°C for 1 h under dry N₂, subsequently 50°C for 1 h under moist N₂, subsequently 100°C for 0.5 h under dry N₂.

The dimerization conditions were the same as in comparative experiment 1.

**Table 4**

| Catalysts (Si/Al ratio) | Isobutene conversion / % | Selectivity / % | | | |
|---|---|---|---|---|---|
| | | C₈ | | C₁₂ | C_{16&+} |
| | | 2,4,4-Trimethyl pentene | Others | | |
| H-ZSM-5 (27.5) | 72.8 | 39.4 | 0.2 | 36.8 | 23.6 |
| H-ZSM-5 (45) | 47.0 | 88.9 | 0.6 | 8.4 | 2.1 |
| H-Mordenite (20) | 87.4 | 44.3 | 3.9 | 41.3 | 10.5 |
| H-Beta (20.5) | 83.0 | 31.4 | 0.0 | 44.5 | 24.1 |
| H-Y (6) | 68.2 | 34.0 | 0.1 | 19.6 | 46.3 |
| H-SAPO-5 (0.4) | 18.5 | 83.3 | 0 | 16.7 | 0.0 |

Comparative experiment 1 in which moist gas is not used results in low C8 selectivity. Comparison of Tables 1 and 4 shows that the contacting with an inert gas comprising water vapour results in a higher selectivity of the desired 2,4,4-trimethyl pentene.

### Example 2

H-ZSM-5 zeolite (Si/Al ratio 45) catalysts pre-treated at different conditions were used for isobutene dimerization.

Pre-calcined samples were pre-treated at various conditions including the contacting step with moist N2. The duration of the contacting step with dry N2 before the contacting step with moist N2 was varied as shown in Table 5.

The feed and the dimerization conditions were the same as in comparative experiment 1.

**Table 5:**

| Moisture pre-treatment conditions / Setp-1/Step-2/Step-3 | Isobutene conversion /% | Selectivity / % | | | |
|---|---|---|---|---|---|
| | | C₈ | | C₁₂ | C_{16&+} |
| | | 2,4,4-Trimethyl pentene | Others | | |
| 300°C for 0.5 h under dry N₂/ 50°C for 1 h under moist N₂/ 100°C for 0.5 h under dry N₂ | 38.8 | 92.8 | 0.0 | 6.2 | 1.0 |
| 300°C for 1 h under dry N₂/ 50°C for 1 h under moist N₂/ 100°C for 0.5 h under dry N₂ | 47.0 | 88.9 | 0.6 | 8.4 | 2.1 |
| 300°C for 1.5 h under dry N₂/ 50°C for 1 h under moist N₂/ 100°C for 0.5 h under dry N₂ | 47.4 | 87.4 | 0.6 | 9.7 | 2.3 |

### Example 3

H-ZSM-5 zeolite (Si/Al ratio 45) catalysts pre-treated at different conditions were used for isobutene dimerization.

Pre-calcined samples were pre-treated at various conditions including the contacting step with moist N2. The duration of the contacting step with moist N2 was varied as shown in Table 6.

The feed and the dimerization conditions were the same as in comparative experiment 1.

**Table 6**

| Moisture pre-treatment period / h | Isobutene conversion /% | Selectivity / % | | | |
|---|---|---|---|---|---|
| | | C₈ | | C₁₂ | C_{16 & +} |
| | | 2,4,4-Trimethyl pentene | Others | | |
| 300°C for 1 h under dry N₂/ 50°C for 0.5 h under moist N₂/ 100°C for 0.5 h under dry N₂ | 84.8 | 82.6 | 0.5 | 16.1 | 0.8 |
| 300°C for 1 h under dry N₂/ 50°C for 1 h under moist N₂/ 100°C for 0.5 h under dry N₂ | 47.0 | 88.9 | 0.6 | 8.4 | 2.1 |
| 300°C for 1 h under dry N₂/ 50°C for 1.5 h under moist N₂/ 100°C for 0.5 h under dry N₂ | 45.1 | 89.2 | 0.5 | 9.0 | 1.3 |

### Example 4

H-ZSM-5 zeolite (Si/Al ratio 45) catalysts pre-treated at different conditions were used for isobutene dimerization.

Pre-calcined samples were pre-treated at various conditions including the contacting step with moist N2. The temperature of the contacting step with dry N2 after the contacting step with moist N2 was varied as shown in Table 7. The feed and the dimerization conditions were the same as in comparative experiment 1.

**Table 7**

| Moisture removal temperature / °C | Isobutene conversion /% | Selectivity / % | | | |
|---|---|---|---|---|---|
| | | C₈ | | C₁₂ | C_{16&+} |
| | | 2,4,4-Trimethyl pentene | Others | | |
| 300°C for 1 h under dry N₂/ 50°C for 1 h under moist N₂/ 75°C for 0.5 h under dry N₂ | 42.4 | 93.0 | 0.8 | 4.6 | 1.6 |
| 300°C for 1 h under dry N₂/ 50°C for 1 h under moist N₂/ 100°C for 0.5 h under dry N₂ | 47.0 | 88.9 | 0.6 | 8.4 | 2.1 |
| 300°C for 1 h under dry N₂/ 50°C for 1.5 h under moist N₂/ 125°C for 0.5 h under dry N₂ | 78.2 | 82.4 | 0.5 | 15.8 | 1.3 |

### Example 5

H-ZSM-5 zeolite (Si/Al ratio 45) catalysts pre-treated at different conditions were used for isobutene dimerization.

Pre-calcined samples were pre-treated at various conditions including the contacting step with moist N2. The duration of the contacting step with dry N2 after the contacting step with moist N2 was varied as shown in Table 8.

The feed and the dimerization conditions were the same as in comparative experiment 1.

**Table 8:**

| Moisture removal period / h | Isobutene conversion /% | Selectivity / % | | | |
|---|---|---|---|---|---|
| | | C8 | | C₁₂ | C_{16 & +} |
| | | 2,4,4-Trimethyl pentene | Others | | |
| 300°C for 1 h under dry N₂/ 50°C for 1 h under moist N₂/ 100°C for 0.25 h under dry N₂ | 34.3 | 90.0 | 0.8 | 7.6 | 1.6 |
| 300°C for 1 h under dry N₂/ 50°C for 1 h under moist N₂/ 100°C for 0.5 h under dry N₂ | 47.0 | 88.9 | 0.6 | 8.4 | 2.1 |
| 300°C for 1 h under dry N₂/ 50°C for 1 h under moist N₂/ 100°C for 0.75 h under dry N₂ | 55.0 | 88.1 | 0.8 | 11.1 | 0.0 |
| 300°C for 1 h under dry N₂/ 50°C for 1 h under moist N₂/ 100°C for 1 h under dry N₂ | 64.2 | 87.6 | 0.5 | 11.5 | 0.4 |

### Example 6

Effect of reaction temperature on the performance of H-ZSM-5 zeolite (Si/Al ratio of 45) for isobutene dimerization is shown in table 9.

The feed and the reaction conditions, except temperature, were the same as in comparative experiment 1.

Catalyst pre-treatment: Pre-calcined sample was treated at 300°C for 1 h under dry N₂/ 50°C for 1 h under moist N₂/ 100°C for 0.5 h under dry N₂.

**Table 9:**

| Reaction temperature / °C | Isobutene conversion / % | Selectivity / % | | | |
|---|---|---|---|---|---|
| | | C₈ | | C₁₂ | C_{16 & +} |
| | | 2,4,4-Trimethyl pentene | Others | | |
| 50 | 47.0 | 88.9 | 0.6 | 8.4 | 2.1 |
| 60 | 52.0 | 84.2 | 0.8 | 14.2 | 0.8 |
| 70 | 78.6 | 63.9 | 0.3 | 29.3 | 6.5 |

### Example 7

Effect of feed composition on the performance of H-ZSM-5 zeolite (Si/Al ratio of 45) for isobutene dimerization is shown in table 10.

Reaction conditions were the same as in comparative experiment 1.

Catalyst pre-treatment: Pre-calcined sample was treated at 300°C for 1 h under dry N₂/ 50°C for 1 h under moist N₂/ 100°C for 0.5 h under dry N₂.

**Table 10:**

| Isobutene/Isobutane / Weight ratio | Isobutene conversion /% | Selectivity / % | | | |
|---|---|---|---|---|---|
| | | C₈ | | C₁₂ | C_{16 & +} |
| | | 2,4,4-Trimethyl pentene | Others | | |
| 40 : 60 | 47.9 | 90.8 | 0.5 | 8.7 | 0.0 |
| 50 : 50 | 47.0 | 88.9 | 0.6 | 8.4 | 2.1 |
| 60 : 40 | 71.5 | 73.3 | 3.3 | 19.7 | 3.7 |

## Claims

1. Method for pre-treating a catalyst composition comprising contacting a medium pore aluminosilicate zeolite with an inert gas comprising water vapour or alcohol vapour at a temperature between 30 °C and the boiling temperature of water or the alcohol, preferably 30-90 °C, more preferably 40-70 °C.

2. The method according to claim 1, wherein the alcohol is selected from the group consisting of i-propanol, isopropanol, isobutanol and tertiary butanol or a mixture thereof.

3. The method according to claim 1 or 2, wherein the inert gas is selected from the group consisting of nitrogen, helium and argon or a mixture thereof.

4. The method according to any one of claims 1-3, wherein the zeolite is a 10-ring zeolite having pores formed by a ring consisting of 10 SiO₄ tetrahedra.

5. The method according to any one of claims 1-4, wherein the zeolite has a pore size of 5-6 Å and preferably a pore size of 5.2-5.8 A.

6. The method according to any one of claims 1-5, the zeolite is of the ZSM-5 type.

7. The method according to any one of claims 1-6, wherein the silicon to aluminium (Si:Al) molar ratio of the zeolite is 10-100, preferably 30-60 and most preferably 40-50.

8. The method according to any one of claims 1-7, wherein the zeolite comprises up to 1 wt% of one or more elements selected from Groups 6 and 9 of the Periodic Table.

9. The method according to claim 8, wherein said one or more elements are selected from the group consisting of molybdenum, tungsten, cobalt and rhodium.

10. Process for the dimerization of olefins or a mixture of olefins and paraffins, said olefins and paraffins having between 2 to 8 carbon atoms, comprising:
(a) pre-treating a catalyst composition according to the method of any one of claims 1-9; and
(b) contacting the treated catalyst composition with a feedstream comprising said olefins or a mixture of olefins and paraffins.

11. The.process according to claim 10, wherein the molar ratio of the olefins to paraffins in the feedstream is 1:0.1-10, preferably 1:0.2-5, and most preferably 1:0.5-2.

12. The process according to claim 10 or 11, wherein the feedstream comprises isobutene and isobutane.

13. The process according to any one of claims 11-13, wherein the pre-treated catalyst composition is contacted with a feedstream under conditions comprising a temperature of 40-80 °C and a pressure of 1-2.5 MPa.

14. The pre-treated catalyst composition obtainable by the method according to any one of claims 1-9.
